# EUROPEAN PATENT APPLICATION

(11) **EP 1 193 267 A1**
(43) Date of publication of application: **03.04.2002**
(21) Application number: 00810886.2
(22) Date of filing: 27.09.2000
(51) Int. Cl.: C07D 409/12, A61K 31/4535, A61P 9/10, A61P 19/02, A61P 25/00, A61P 35/00

(54) **Pharmaceutically active hydrophilic sulfonamide derivatives as inhibitors of protein JunKinases**

(71) Applicant: Applied Research Systems ARS Holding N.V., Curacao (AN)
(72) Inventor: Halazy, Serge, 74100 Vetraz-Monthoux (FR); Church, Dennis, 1291 Commugny (CH); Camps, Montserrat, 1290 Versoix (CH); Rueckle, Thomas, 1228 Plan-Les-Ouates (CH); Gotteland, Jean Pierre, 74160 Beaumont (FR); Biamonte, Marco, 1227 Carouge (CH); Arkinstall, Stephen, Belmont, MA 02478 (US)
(74) Representative: Moinas, Michel

(57) **Abstract**

The present invention is related to substantially hydrophilic sulfonamide derivatives, or sulfonamide derivatives having a substantially hydrophilic moiety, of formula I notably for use as pharmaceutically active compounds, as well as to pharmaceutical formulations containing such sulfonamide derivatives. Said sulfonamide derivatives are efficient modulators of the JNK pathway, they are in particular efficient and selective inhibitors of JNK 2 and 3. The present invention is furthermore related to novel sulfonamide derivatives as well as to methods of their preparation. 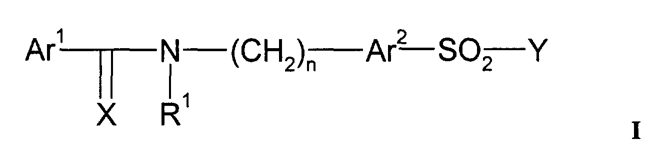 The compounds of formula I according to the present invention being suitable pharmaceutical agents are those wherein
Ar¹ is a substituted or unsubstituted aryl or heteroaryl;
Ar² is an aryl or heteroaryl group carrying at least one hydrophilic substituent;
X is O or S, preferably O;
R¹ is hydrogen or a C₁-C₆-alkyl group, or R¹ forms a substituted or unsubstituted 5-6-membered saturated or unsaturated ring with Ar¹;
n is an integer from 0 to 5, preferably between 1-3 and most preferred 1;
Y within formula I is an unsubstituted or a substituted 4-12-membered saturated cyclic or bicyclic alkyl containing at least one nitrogen atom, whereby one nitrogen atom within said ring is forming a bond with the sulfonyl group of formula I thus providing a sulfonamide.

## Description

### Field of the invention

The present invention is related to substantially hydrophilic sulfonamide derivatives or sulfonamide derivatives having a substantially hydrophilic moiety for use as pharmaceutically active compounds, as well as pharmaceutical formulations containing such sulfonamide derivatives. In particular, the present invention is related to sulfonamide derivatives displaying a substantial modulatory, notably an inhibitory activity of the JNK (JunKinase) function or pathways respectively, and which are therefore particularly useful in the treatment and/or prevention of disorders of the autoimmune and the neuronal system. The present invention is furthermore related to novel sulfonamide derivatives as well as to methods of their preparation.

### Background of the invention

Apoptosis denotes the complex contortions of the membrane and organelles of a cell as it undergoes the process of programmed cell death. During said process, the cell activates an intrinsic suicide program and systematically destroys itself. The following series of events can be observed :
- The cell surface begins to bleb and expresses pro-phagocytic signals. The whole apoptotic cell then fragments into membrane-bound vesicles that are rapidly and neatly disposed of by phagocytosis, so that there is minimal damage to the surrounding tissue.
- The cell then separates from its neighbors.

The nucleus also goes through a characteristic pattern of morphological changes as it commits genetic suicide, the chromatin condenses and is specifically cleaved to fragments of DNA.

Neuronal cell death plays an important role in ensuring that the nervous system develops normally. It appears that the death of developing neurons depends on the size of the target that they innervate: cells with fewer synaptic partners are more likely to die than those that have formed multiple synapses. This may reflect a process, which balances the relative number of pre- to postsynaptic neurons in the developing nervous system. Although neuronal cell death was assumed to be apoptotic, it was only recently that neurons in developing rodent brain were conclusively shown to undergo apoptosis as classified by morphology and DNA fragmentation. As cell death during development is clearly not a pathological process, it makes sense that cells actually cease to exist. Neuronal death occurs via either apoptotic or necrotic processes following traumatic nerve injury or during neurodegenerative diseases. Multiple components are emerging as key players having a role in driving neuronal programmed cell death. Amongst the components leading to neuronal apoptosis are members of the SAPK/JNK being a sub-family of MAP Kinases (MAPKs).

MAPKs (mitogen-activated protein kinases) are serine/threonine kinases that are activated by dual phosphorylation on threonine and tyrosine residues. In mammalian cells, there are at least three separate but parallel pathways that convey information generated by extra-cellular stimuli to the MAPKs. Said pathways consist of kinase cascades leading to activa-tion of the ERKs (extracellular regulated kinases), the JNKs (c-Jun N-terminal kinases), and the p38/CSBP kinases. While both the JNK and p38 pathways are involved in relaying stress-type extramolecular signals, the ERK pathway is primarily responsible for transducing mitogenic/differentiation signals to the cell nucleus. SAPK cascades represent a sub-family of the mitogen-activating protein kinase family, that are activated by different external stimuli including DNA damage following UV irradia-tion, TNF-α, IL-1β, ceramide, cellular stress, and reactive oxygen species and have dis-tinct substrate specificities. Signal transduction via MKK4/JNK of MKK3/p38 results in the phosphorylation of inducible transcription factors, c-Jun and ATF2, which then act as either homodimers or heterodimers to initiate transcription of down-stream effectors.

c-Jun is a protein that is forming homodimers and heterodimers (with e.g. c-Fos) to produce the transactivating complex AP-which is required for the activation of many genes (e.g. matrix metalloproteinases) involved in the inflammatory response. The JNKs were discovered when it was found that several different stimuli such as UV light and TNF-α stimulated phosphorylation of c-Jun on specific serine residues in the N-terminus of the protein.

In a recent publication of Xie X et al, *(Structure* **1998,** *6 (8);* 983-991) it has been suggested that activation of stress-activated signal transduction pathways are required for neuronal apoptosis induced by NGF withdrawal in rat PC-12 and superior cervical ganglia (SCG) sympathetic neuronal cells. Inhibition of specific kinases, namely MAP kinase kinase 3 (MKK3) and MAP kinase kinase 4 (MKK4), or c-Jun (part of the MKK-4 cascade) may be sufficient to block apoptosis (see also Kumagae Y et al, in *Brain Res Mol Brain Res,* **1999,** *67(1),* 10-17 and Yang DD et al in *Nature*, **1997,** *389 (6653)*; 865-870). Within a few hours of NGF deprivation in SCG neurones, c-Jun becomes highly phosphorylated and protein levels increase. Similarly in rat PC-12 cells deprived of NGF, JNK and p38 undergo sustained activation while ERKs are inhibited. Consistent with this JNK3 KO mice are resistant to excitotoxicity induced apoptosis in the hippocampus and more importantly they display greatly reduced epileptic like seizures in response to excitotoxicity as compared to normal animals *(Nature* **1997,** *389,* 865-870). More recently, it has been reported that the JNK signalling pathway is implicated in cell proliferation and could play an important role in autoimmune diseases *(Immunity,* **1998,** *9*, 575-585; *Current Biology*, **1999,** *3*, 116-125) which are mediated by T-cell activation and proliferation.

Naive (precursor) CD4⁺ helper T (Th) cells recognise specific MHC-peptide complexes on antigen-presenting cells (APC) via the T-cell receptor (TCR) complex. In addition to the TCT-mediated signal, a co-stimulatory signal is provided at least partially by the ligation of CD28 expressed on T-cells with B7 proteins on APC. The combination of these two signals induces T-cell clonal expression.

After 4-5 days of proliferation, precursor of CD4⁺ T cells differentiate into armed effector Th cells that mediate the functions of the immune system. During the differentiation process, substantial reprogramming of gene expression occurs.

Two subsets of effector Th cells have been defined on the basis of their distinct cytokine secretion pattern and their immuno-modulatory effects: Th1 cells produce IFNγ and LT (TNF-β), which are required for cell-mediated inflammatory reactions; Th2 cells secrete IL-4, IL-5, IL-6, IL-10 and IL-13, which mediate B cell activation and differentiation. These cells play a central role in the immune response. The JNK MAP Kinase pathway is induced in Th1 but not in Th2 effector cells upon antigen stimulation. Furthermore, the differentiation of precursor CD4⁺ T cells into effector Th1 but not Th2 cells is impaired in JNK2-deficient mice. Therefore, in recent years it has been realised that the JNK kinase pathway plays an important role in the balance of Th1 and Th2 immune response through JNK2.

With the objective of inhibiting the JNK kinase pathway, WO/9849188 teaches the use of a human polypeptide, i.e. JNK-interacting protein 1 (JIP-1), which is a biological product and which has also been assayed for overcoming apoptosis related disorders. Although such human polypeptides have been confirmed to have an inhibitory effect onto the JNK kinase pathway, a whole variety of drawbacks are associated with their use :
- Active bio-peptides or bio-proteins are only obtained by means of rather comprehensive and expensive bio-synthesis which consequently frequently renders the resulting products fairly cost-intensive.
- The peptides are known to display poor membrane penetration and may not cross the blood brain membrane,
- The principal drawback to the use of peptide inhibitors or antagonists is the problem of low oral bioavailability resulting from intestinal degradation. Hence, they must be administered parenterally and finally,
- peptide inhibitors or antagonists are frequently viewed by the host body as intruding material to be eliminated, thus setting off an auto-immune response.

Hence, it is an objective of the present invention to provide relatively small molecules that avoid essentially all of the above-mentioned drawbacks arising from the use of peptides or proteins, however, which are suitable for the treatment of a variety of diseases, in particular of neuronal or the autoimmune system related disorders. It is notably an objective of the present invention to provide relatively small molecule chemical compounds which are able to modulate, preferably to down-regulate or to inhibit the JNK (Jun kinase) pathway so to be available as a convenient method of treating diseases which are preferably mediated by the JNK function. Moreover, it is an objective of the present invention to provide methods for preparing said small molecule chemical compounds. It is furthermore an objective of the present invention to provide a new category of pharmaceutical formulations for the treatment of diseases, preferably mediated by the JNK function. It is finally an objective of the present invention to provide a method for the treatment and/or prevention of diseases that are caused by disorders of the autoimmune and/or the neuronal system.

### Description of the invention

The aforementioned objectives have been met according to the independent claims. Preferred embodiments are set out within the dependent claims which are incorporated herewith.

The following paragraphs provide definitions of the various chemical moieties that make up the compounds according to the invention and are intended to apply uniformly throughout the specification and claims unless an otherwise expressly set out definition provides a broader definition.

"C₁-C₆-alkyl" refers to monovalent alkyl groups having 1 to 6 carbon atoms. This term is exemplified by groups such as methyl, ethyl, n-propyl, isopropyl, n-butyl, isobutyl, tert-butyl, n-hexyl and the like.

"Aryl" refers to an unsaturated aromatic carbocyclic group of from 6 to 14 carbon atoms having a single ring (e.g. phenyl) or multiple condensed rings (e.g. naphthyl). Preferred aryl include phenyl, naphthyl, phenantrenyl and the like.

"C₁-C₆-alkyl aryl" refers to C₁-C₆-alkyl groups having an aryl substituent, including benzyl, phenethyl and the like.

"Heteroaryl" refers to a monocyclic heteroaromatic, or a bicyclic or a tricyclic fused-ring heteroaromatic group. Particular examples of heteroaromatic groups include optionally substituted pyridyl, pyrrolyl, furyl, thienyl, imidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl,1,3,4-triazinyl, 1,2,3-triazinyl, benzofuryl, [2,3-dihydro]benzofuryl, isobenzofuryl, benzothienyl, benzotriazolyl, iso-benzothienyl, indolyl, isoindolyl, 3H-indolyl, benzimidazolyl, imidazo[1,2-a]pyridyl, benzothiazolyl, benzoxazolyl, quinolizinyl, quinazolinyl, pthalazinyl, quinoxalinyl, cinnnolinyl, napthyridinyl, pyrido[3,4-b]pyridyl, pyrido[3,2-b]pyridyl, pyrido[4,3-b]pyridyl, quinolyl, isoquinolyl, tetrazolyl, 5,6,7,8-tetrahydroquinolyl, 5,6,7,8-tetrahydroisoquinolyl, purinyl, pteridinyl, carbazolyl, xanthenyl or benzoquinolyl.

"C₁-C₆-alkyl heteroaryl" refers to C₁-C₆-alkyl groups having a heteroaryl substituent, including 2-furylmethyl, 2-thienylmethyl, 2-(1H-indol-3-yl)ethyl and the like.

"Alkenyl" refers to alkenyl groups preferably having from 2 to 6 carbon atoms and having at least 1 or 2 sites of alkenyl unsaturation. Preferable alkenyl groups include ethenyl (-CH=CH₂), n-2-propenyl (allyl, -CH₂CH=CH₂) and the like.

"Alkynyl" refers to alkynyl groups preferably having from 2 to 6 carbon atoms and having at least 1-2 sites of alkynyl unsaturation, preferred alkynyl groups include ethynyl (-C≡CH), propargyl (-CH₂C≡CH), and the like.

"Acyl" refers to the group -C(O)R where R includes "C₁-C₆-alkyl", "aryl", "heteroaryl", "C₁-C₆-alkyl aryl" or "C₁-C₆-alkyl heteroaryl".

"Acyloxy" refers to the group -OC(O)R where R includes "C₁-C₆-alkyl", "aryl", "heteroaryl", "C₁-C₆-alkyl aryl" or "C₁-C₆-alkyl heteroaryl".

"Alkoxy" refers to the group -O-R where R includes "C₁-C₆-alkyl" or "aryl" or "heteroaryl" or "C₁-C₆-alkyl aryl" or "C₁-C₆-alkyl heteroaryl". Preferred alkoxy groups include by way of example, methoxy, ethoxy, phenoxy and the like.

"Alkoxycarbonyl" refers to the group -C(O)OR where R includes "C₁-C₆-alkyl" or "aryl" or "heteroaryl" or "C₁-C₆-alkyl aryl" or "C₁-C₆-alkyl heteroaryl".

"Aminocarbonyl" refers to the group -C(O)NRR' where each R, R' includes independently hydrogen or C₁-C₆-alkyl or aryl or heteroaryl or "C₁-C₆-alkyl aryl" or "C₁-C₆-alkyl heteroaryl".

"Acylamino" refers to the group -NR(CO)R' where each R, R' is independently hydrogen or "C₁-C₆-alkyl" or "aryl" or "heteroaryl" or "C₁-C₆-alkyl aryl" or "C₁-C₆-alkyl heteroaryl".

"Halogen" refers to fluoro, chloro, bromo and iodo atoms.

"Sulfonyl" refers to group "-SO₂-R" wherein R is selected from H, "aryl", "heteroaryl", "C₁-C₆-alkyl", "C₁-C₆-alkyl" substituted with halogens e.g. an -SO₂-CF₃ group, "C₁-C₆-alkyl aryl" or "C₁-C₆-alkyl heteroaryl".

"Sulfoxy" refers to a group "-S(O)-R" wherein R is selected from H, "C₁-C₆-alkyl", "C₁-C₆-alkyl" substituted with halogens e.g. an -SO-CF₃ group, "aryl", "heteroaryl" , "C₁-C₆-alkyl aryl" or "C₁-C₆-alkyl heteroaryl".

"Thioalkoxy" refers to groups -S-R where R includes "C₁-C₆-alkyl" or "aryl" or "heteroaryl" or "C₁-C₆-alkyl aryl" or "C₁-C₆-alkyl heteroaryl". Preferred thioalkoxy groups include thiomethoxy, thioethoxy, and the like.

"Substituted or unsubstituted" : Unless otherwise constrained by the definition of the individual substituent, the above set out groups, like "alkyl", "alkenyl", "alkynyl", "aryl" and "heteroaryl" etc. groups can optionally be substituted with from 1 to 5 substituents selected from the group consisting of "C₁-C₆-alkyl", "C₁-C₆-alkyl aryl", "C₁-C₆-alkyl heteroaryl", "C₂-C₆-alkenyl", "C₂-C₆-alkynyl", primary, secondary or tertiary amino groups or quarter-nary ammonium moieties, "acyl", "acyloxy", "acylamino", "aminocarbonyl", "alkoxycarbonyl", "aryl", "heteroaryl", carboxyl, cyano, halogen, hydroxy, mercapto, nitro, sulfoxy, sulfonyl, alkoxy, thioalkoxy, trihalomethyl and the like. Alternatively said substitution could also comprise situations where neighboring substituents have undergone ring closure, notably when viccinal functional substituents are involved, thus forming e.g. lactams, lactons, cyclic anhydrides, but also acetals, thio-acetals, aminals formed by ring closure for instance in an effort to obtain a protective group.

"Pharmaceutically acceptable salts or complexes" refers to salts or complexes of the below-identified compounds of formula I that retain the desired biological activity. Examples of such salts include, but are not restricted to acid addition salts formed with inorganic acids (e.g. hydrochloric acid, hydrobromic acid, sulfuric acid, phosphoric acid, nitric acid, and the like), and salts formed with organic acids such as acetic acid, oxalic acid, tartaric acid, succinic acid, malic acid, fumaric acid, maleic acid, ascorbic acid, benzoic acid, tannic acid, pamoic acid, alginic acid, polyglutamic acid, naphthalene sulfonic acid, naphthalene disulfonic acid, and polygalacturonic acid. Said compounds can also be administered as pharmaceutically acceptable quaternary salts known by a person skilled in the art, which specifically include the quarternary ammonium salt of the formula -NR,R',R" ⁺ Z⁻, wherein R, R', R" is independently hydrogen, alkyl, or benzyl, and Z is a counterion, including chloride, bromide, iodide, -O-alkyl, toluenesulfonate, methylsulfonate, sulfonate, phosphate, or carboxylate (such as benzoate, succinate, acetate, glycolate, maleate, malate, fumarate, citrate, tartrate, ascorbate, cinnamoate, mandeloate, and diphenylacetate). Sample based-addition salts include those derived from sodium, potassium, ammonium, and quaternary ammonium hydroxide, such as for example tetramethylammonium hydroxide.

"Pharmaceutically active derivative" refers to any compound that upon administration to the recipient, is capable of providing directly or indirectly, the activity disclosed herein.

"Ionisable moiety" refers to functional groups, wherein its characteristic electron distribution confers to said moiety its capacity to be transformed into an ionic or ionised group, e.g. a salt. Such groups may be basic moieties that could be protonated or acidic groups that may be deprotonated. Preferred ionisable moieties are basic groups like amines or acid groups like carboxylic acids.

"Essentially soluble under physiological conditions" means that the compounds of the present invention display for the purpose of achieving a pharmacological effect, under a physiological pH, being at about 7.4, have a sufficient solubility. A preferred threshold is at about 50 µg/mL solvent, more preferably of at least 100 µg/mL solvent.

"Lipophilic chain" refers to groups which have a pronounced attraction to hydrophobic groups, substituents or compounds, notably to lipids or fatty compounds or moieties. They notably include optionally substituted C₄-C₁₈-alkyl groups or a substituted or unsubstituted alkyl-aryl group.

"Hydrophilic group" refers to functional groups which have a pronounced attraction to hydrophilic or polar groups, substituents or compounds or fatty compounds or moieties. They notably include carboxylates, hydroxides, sulfates or sulfonates or amines or ammonium salts.

"Enantiomeric excess" (ee) refers to the products that are obtained by an essentially enantiomeric synthesis or a synthesis comprising an enantioselective step, whereby a surplus of one enantiomer in the order of at least about 52% ee is yielded. In the absence of an enantiomeric synthesis, racemic products are usually obtained that do however also have the inventive set out activity as JunK2 and/or 3 inhibitors.

Quite surprisingly, it was now found that sulfonamide derivatives according to formula I are suitable pharmaceutically active agents, by effectively modulating, in particular by down-regulating inhibiting the action of JNK's, notably of JNK 1 and/or 2 and/or 3. 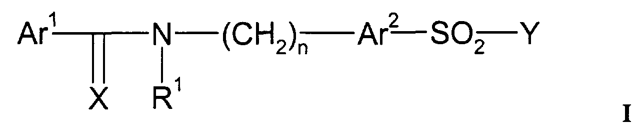 The compounds of formula I according to the present invention being suitable pharmaceutical agents are those wherein
Ar¹ is a substituted or unsubstituted aryl or heteroaryl.
Ar² is an aryl or heteroaryl group carrying at least one hydrophilic substituent.
X is O or S, preferably O.
R¹ is hydrogen or a C₁-C₆-alkyl group, or R¹ forms a substituted or unsubstituted 5-6-membered saturated or unsaturated ring with Ar¹.
n is an integer from 0 to 5, preferably between 1-3 and most preferred 1.
Y within formula I is an unsubstituted or a substituted 4-12-membered saturated cyclic or bicyclic alkyl containing at least one nitrogen atom, whereby one nitrogen atom within said ring is forming a bond with the sulfonyl group of formula I thus providing the sulfonamide.

In a preferred embodiment of the present invention, Y is a piperidine or piperazine moiety according to the below formula

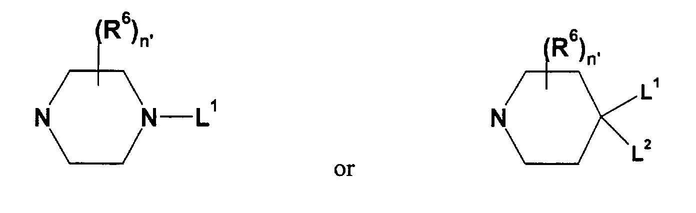

In said piperidine or piperazine groups, L¹ and L²are independently selected from each other from the group comprising or consisting of H, substituted or unsubstituted C₁-C₆-alkyl, substituted or unsubstituted C₂-C₆-alkenyl, substituted or unsubstituted C₂-C₆-alkynyl, substituted or unsubstituted cyclic C₄-C₈-alkyl optionally containing 1-3 heteroatoms and optionally fused with aryl or heteroaryl; or L¹ and L² are independently selected from the group comprising or consisting of substituted or unsubstituted aryl, substituted or unsubstituted heteroaryl, aryl-C₁-C₆-alkyl, heteroaryl-C₁-C₆-alkyl, -C(O)-OR³, -C(O)-R³, -C(O)-NR^{3'}R³, -NR^{3'}R³, -NR^{3'}C(O)R³, -NR^{3'}C(O)NR^{3'}R³, -(SO)R³,-(SO₂)R³, -NSO₂R³, -SO₂NR^{3'}R³.

Thereby, R³ and R^{3'} are substituents independently selected from the group comprising or consisting of H, substituted or unsubstituted C₁-C₆-alkyl, substituted or unsubstituted C₂-C₆-alkenyl, substituted or unsubstituted aryl, substituted or unsubstituted heteroaryl, substituted or unsubstituted aryl-C₁-C₆-alkyl, substituted or unsubstituted heteroaryl-C₁-C₆-alkyl.

R⁶ is selected from the group comprising or consisting of hydrogen, substituted or unsubstituted C₁-C₆-alkyl, substituted or unsubstituted C₁-C₆-alkoxy, OH, halogen, nitro, cyano, sulfonyl, oxo (=O), sulfoxy, acyloxy, thioalkoxy and n' is an integer from 0 to 4, preferably 1 or 2.

All of the above mentioned aryl or heteroaryl groups could optionally be substituted by at least one of the groups selected from substituted or unsubstituted C₁-C₆-alkyl,like trihalomethyl, substituted or unsubstituted C₁-C₆-alkoxy, acyloxy, substituted or unsubstituted C₂-C₆-alkenyl, substituted or unsubstituted C₂-C₆-alkynyl, amino, acylamino, aminocarbonyl, C₁-C₆-alkoxycarbonyl, aryl, carboxyl, cyano, halogen, hydroxy, nitro, sulfonyl, sulfoxy, C₁-C₆-thioalkoxy.

Also L¹ and L² taken together could form a 4-8-membered saturated cyclic alkyl or heteroalkyl group, like triazolines, tetrazolines, oxazolines, isoxazolines, oxazoles or isoxazoles. In a preferred embodiment L¹ and L² form together 5-6-membered saturated cyclic alkyl ring containing 2-3 nitrogen atoms.

L¹ could also be an ionisable moiety to which a lipophilic chain is attached. Such an ionisable moiety could be an amino group which is substituted with a lipophilic C₄-C₁₈ alkyl, preferably C₆-C₁₂ alkyl, or a substituted or unsubstituted alkyl-aryl group.

The above mentioned ionisable moieties within L¹ are meant to confer a better solubility to the molecules of formula I under physiological conditions. The improvement of the solubility of the molecules of formula I through an ionisable moiety within L¹ is of particular interest notably for pharmaceutical compounds which - upon delivery - are to be brought into solution under physiological conditions. The most preferred ionisable moiety is an amino group.

Particularly potent compounds of formula I in respect of the inhibition of JNK3 and/or 2 are those where L¹ also comprises a lipophilic moiety. Most preferred is a C₄-C₁₈ alkyl group attached to an ionisable moiety like an amino group. Such lipophilic groups are believed to enter into a cavity of the enzyme to be inhibited.

The present invention also includes the geometrical isomers, the optical active forms, enantiomers, diastereomers of compounds according to formula I, as well as their racemates and also pharmaceutically acceptable salts as well as the pharmaceutically active derivatives of the sulfonamide derivatives of formula I.

Preferred Ar¹ in formula I are those that are independently selected from the group comprising or consisting of phenyl, thienyl, furyl, pyridyl, optionally substituted by substituted or unsubstituted C₁-C₆-alkyl, like trihalomethyl, substituted or unsubstituted C₁-C₆-alkoxy, substituted or unsubstituted C₂-C₆-alkenyl, substituted or unsubstituted C₂-C₆-alkynyl, amino, acylamino, aminocarbonyl, C₁-C₆-alkoxycarbonyl, aryl, carboxyl, cyano, halo, hydroxy, nitro, sulfonyl, sulfoxy, acyloxy, C₁-C₆- thioalkoxy. The most preferred Ar¹ is a substituted phenyl, e.g. a chlorophenyl, nitrophenyl, hydroxyphenyl, alkoxy phenyl,

The most preferred Ar² is a thienyl, pyrrolo or furanyl group with at least one, preferably one hydrophilic substituent. Such hydrophilic substituents attached to said thienyl, pyrrolo or furanyl group are residues conferring a better solubility to the molecules of formula I. They include notably carboxylic groups, carboxylates, carboxamides, OH, or OH carrying alkyl groups, or hydrazido carbonyl groups. The improvement of the solubility of the molecules of formula I through hydrophilic substituents on Ar² is of particular interest notably for pharmaceutical compounds which are to be brought into solution under physiological conditions.

Particularly preferred sulfonamides are those wherein Ar¹ is a phenyl group, X is O, R¹ is hydrogen, n is 1, Ar² is a thienyl group with one hydrophilic substituent.

A particularly preferred embodiment of the present invention is related to the sulfonamide derivatives of formula I, wherein Y is a substituted or unsubstituted piperidine residue, 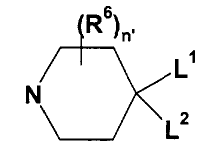 whereby R⁶, n', L¹ and L² are as above defined.

According to a more preferred embodiment, the sulfonamide derivatives according to formula I are those, wherein Ar¹ is 4-chlorophenyl, X is O, R¹ is hydrogen, n is 1, Ar² is thienyl, Y is 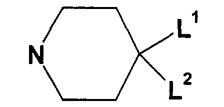 whereby L² is H, L¹ is - -NHR³; with R³ being a substituent selected from the group comprising or consisting of C₁-C₁₂-alkyl, aryl, heteroaryl, aryl-C₁-C₆-alkyl, heteroaryl-C₁-C₆-alkyl.
Said aryl or heteroaryl groups could optionally be substituted by halogen, hydroxy, nitro, sulfonyl.

Specific examples of compounds of formula I include the following :
5-{[(3-methoxybenzoyl)amino]methyl}-2-[(4-{3-[(trifluoromethyl)sulfonyl]anilino}piperidin-1-yl)sulfonyl]thiophene-3-carboxylic acid
5-{[(3-methoxybenzoyl)amino]methyl}-2-{[4-(octylamino)piperidin-1-yl]sulfonyl}thiophene-3-carboxylic acid
N-(2-hydroxyethyl)-5-{[(3-methoxybenzoyl)amino]methyl}-2-[(4-{3-[(trifluoromethyl)sulfonyl]anilino}piperidin-1-yl)sulfonyl]thiophene-3-carboxamide
N-({4-(hydrazinocarbonyl)-5-[(4-{3-[(trifluoromethyl)sulfonyl]anilino}piperidin-1-yl)sulfonyl]thien-2-yl}methyl)-3-methoxybenzamide
5-{[(3-methoxybenzoyl)amino]methyl}-2-[(4-{3-[(trifluoromethyl)sulfonyl]anilino}piperidin-1-yl)sulfonyl]thiophene-3-carboxamide
N-[2-(dimethylamino)ethyl]-5-{[(3-methoxybenzoyl)amino]methyl}-2-[(4-{3-[(trifluoromethyl)sulfonyl]anilino}piperidin-1-yl)sulfonyl]thiophene-3-carboxamide
N-({4-(hydroxymethyl)-5-[(4-{3-[(trifluoromethyl)sulfonyl]anilino}piperidin-1-yl)sulfonyl]thien-2-yl}methyl)-3-methoxybenzamide

A further aspect of the present invention consists in the use of the sulfonamide derivatives according to formula I for the preparation of pharmaceutical compositions for the modulation - notably for the down-regulation, e.g. up to the inhibition - of the JNK function or signalling pathway associated disorders, in particular against neuronal disorders and/or against disorders of the immune system as well as said pharmaceutical compositions themselves. Preferred JNK pathways are the JNK 1 and/or 2 and/or 3.

As above pointed out, the compounds of formula I are suitable to be used as a medicament. Hence, it is herein reported that the compounds falling under the above set out generic formula I are suitable for use in treating disorders of the autoimmune system and neuronal system of mammals, notably of human beings. More specifically, the compounds according to formula I, alone or in the form of a pharmaceutical composition, are useful for the modulation of the JNK pathway, more specifically for treatment or prevention of disorders associated with abnormal expression or activity of JNK, notably of JNK 1 and/or 2 and/or 3. Said modulation usually preferably involves the inhibition of the JNK pathways, notably of the JNK 1 and/or 2 and/or 3. Such an abnormal expression or activity of JNK could be triggered by numerous stimuli (e.g. stress, septic schock, oxidative stress, cytokines) and could lead to out-of-control apoptosis or auto-immune diseases that is frequently involved in the below enumerated disorders and disease states. Hence, the compounds according to formula I could be used for the treatment of disorders by modulating the JNK function or signalling pathways. Said modulation of the JNK function or pathways could involve its activation, but preferably it involves the down-regulation up to inhibition of the JNK pathways, notably of theJNK1 and/or JNK2 and/or 3. The compounds according to formula I could be employed alone or in combination with further pharmaceutical agents, e.g. with a further JNK modulator.

Specifically, the compounds pursuant to formula I are useful for the treatment or prevention of immuno- and/or neuronal-related diseases or pathological states in which inhibition of JNK 1 and/or 2 and/or 3 plays a critical role such as epilepsy; neurodegenerative diseases including Alzheimer's disease, Huntington's disease, Parkinson's disease; retinal diseases; spinal cord injury; head trauma, autoimmune diseases including multiple sclerosis, inflammatory bowel disease (IBD), rheumatoid arthritis; asthma; septic shock; transplant rejection; cancers including breast, colorectal, pancreatic and cardiovascular diseases including stroke, cerebral ischemia, arterosclerosis, myocordial infarction, myocordial reperfusion injury.

Quite surprisingly it turned out that the inventively found compounds according to formula I do show a considerable activity as inhibitors of JNK2 and 3. According to a preferred embodiment, the compounds according to the invention are essentially inactive in view of 2 further apoptosis modulating enzymes, i.e. p38 and/or ERK2, belonging incidentally to the same family as JNK2 and 3. Hence, the compounds according to the present invention offer the possibility to selectively modulate the JNK pathway, and in particular to treat disorders related to the JNK pathways, while being essentially inefficient with regard to other targets like said p38 and ERK2, so that they could indeed be viewed as selective inhibitors. This is of considerable significance, as these related enzymes are generally involved in different disorders, so that for the treatment of a distinct disorder, it is desired to employ a correspondingly selective medicament.

Still a further object of the present invention is a process for preparing the novel sulfonamide derivatives according to formula I which have been set out above. The sulfonamide derivatives of this invention can be prepared from readily available starting materials using the following general methods and procedures. It will be appreciated that where typical or preferred experimental conditions (i.e., reaction temperatures, time, moles of reagents, solvents, etc.) are given, other experimental conditions can also be used unless otherwise stated. Optimum reaction conditions may vary with the particular reactants or solvent used, but such conditions can be determined by one skilled in the art by routine optimisation procedures.

In a preferred method of synthesis, the sulfonamide derivatives of the invention are prepared by first coupling an amine of formula II: 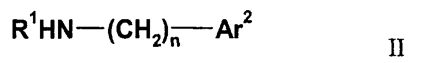 where Ar² and R¹ are as defined above, with an acyl chloride of formula III: 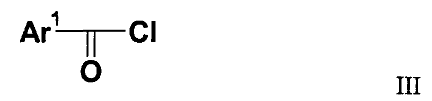 where Ar¹ is as defined above, to provide an amide of formula IV:

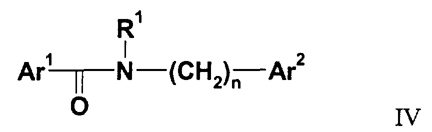

Amines of formula II are either known compounds or can be prepared from known compounds by conventional procedures. Preferred amines as starting materials include thien-2-yl-methylamine, furan-2-yl-methylamine, pyridyl-2-ylmethylamine and the like. The acyl chlorides of formula III are also commercially available or previously described compounds. Preferred acyl chlorides include 4-chlorobenzoyl chloride, 3-methoxybenzoyl chloride, 2-nitrobenzoyl chloride and the like. If not known, the acid halide can be prepared by reacting the corresponding carboxylic acid with an inorganic acid halide, such as thionyl chloride, phosphorus trichloride or oxalyl chloride under conventional conditions.

Generally, this reaction is performed upon using about 1 to 5 molar equivalents of the inorganic acid halide or oxalyl chloride, either in pure form or in an inert solvent, such as carbon tetrachloride, at temperature in the range of about 0°C to about 80°C for about 1 to about 48 hours. A catalyst, as *N,N*-dimethylformamide, may also be used in this reaction.

When an acyl halide is employed in the coupling reaction, it is typically reacted with amine II in the presence of a suitable base to scavenge the acid generated during the reaction. Suitable bases include, by way of example, triethylamine, diisopropylethylamine, *N*-methylmorpholine and the like. Alternatively, an excess of amine II may be used to scavenge the acid generated during the reaction.

Alternatively, the carboxylic acid of compound III can be employed in the coupling reaction. The carboxylic acid of III are usually commercially available reagents or can be prepared by conventional procedures.

The coupling reaction of carboxylic acid of III (i.e. the acyl chloride) is conducted upon using any conventional coupling reagent including, for example, carbodiimides such as dicyclohexylcarbodiimide, N-(3-dimethylaminopropyl)-N'-ethylcarbodiimide and other promoting agents, such as *N,N*-carbonyl-diimidazole or PyBOP. This reaction can be conducted with or without the use of well known additives such as *N*-hydroxysuccinimide, 1-hydroxybenzotriazole, etc. which are known to facilitate the coupling of carboxylic acids and amines.

The coupling reaction using either acid halide III or its carboxylic acid is preferably conducted at a temperature of from about 0°C to about 6°C for about 1 to about 24 hours. Typically, the reaction is conducted in an inert aprotic polar solvent such as N,N-dimethylformamide, dichloromethane, chloroform, acetonitrile, tetrahydrofuran and the like using about 1 to about 5 molar equivalents of the amine based on the carboxylic acid or its acid halide. Upon completion of the reaction, the carboxamide IV is recovered by conventional methods including precipitation, chromatography, filtration, distillation and the like.

The sulfonyl chorides of formula V necessary for the preparation of the sulfonylpiperidines or piperazines of formula I are prepared using conventional sulfonating methods:

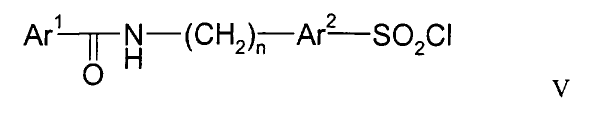

A preferred sulfonating reagent for use in this reaction is chlorosulfonic acid. Typically, the sulfonation reaction is performed by treating the carboxamide of formula (IV) with about 5 to about 10 molar equivalent of the sulfonating reagent in an inert solvent, such as dichloromethane, at a temperature ranging from about -70°C to about 50°C. Preferably, the addition of chlorosulfonic acid takes place at -70°C and leads to the formation of the intermediate sulfonic acid. Increasing the temperature to 20°C allows the formation of the sulfonyl chloride of formula V.

According to a further preferred method of preparation notably in case that the above pointed out method leading to the preliminary synthesis of sulfonyl chloride of formula V is not applicable, the sulfonyl piperidines and piperazines of this invention are prepared by the following steps:
- Protection of the amine function of compounds of formula II;
- Chlorosulfonylation of the aromatic group;
- Formation of the sulfonamide function;
- Deprotection of the protecting group;
- Acylation of the above generated free amine;

Amines of formula II are protected with a suitable protecting group of an amine moiety to provide intermediate of formula VI wherein P denotes the protecting group.

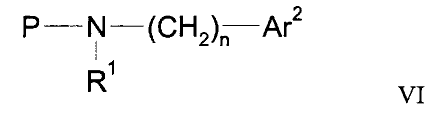

Numerous protecting groups P of the amine function as well as their introduction and removal, are well described in T.W. Greene and G.M. Wuts, Protecting groups in Organic Synthesis, Third Edition, Wiley, New York, 1998, and references cited therein. Preferred are protecting groups that are acids and bases stable and can be further removed by using metal transition complexes such as palladium complexes, for example the allylcarbamate group (Alloc) or the N,N'-bisallyl group. Another preferred protecting group is the maleimide group which is stable in a all range of experimental conditions.

The introduction of said groups can be performed by reacting the corresponding bisallylcarbonate anhydride or allylbromide or maleic anhydride in the presence of a base such as triethylamine, diisopropylethylamine, *N*-methylmorpholine and the like in an aprotic solvent such as N,N-dimethylformamide, dichloromethane, chloroform, acetonitrile, tetrahydrofuran and the like at a temperature ranging from about 0°C to about 80°C.

Compounds of formula VI are then sulfonated using a conventional very mild sulfonating procedure that allows the obtention of sulfonyl chloride of formula VII.

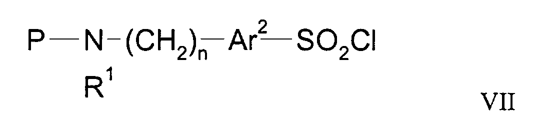

Typically, protected amine VI is treated with a base such as n-butyllithium or tert-butyllithium under an inert atmosphere, in a polar aprotic solvent such as tetrahydrofuran, ether or dioxane at a temperature ranging from -70°C to 0°C during a time ranging from 15 minutes to 4 hours. The so formed anion is then treated with SO₂Cl₂ or most preferably SO₂ by bubbling the gas into the reaction mixture at a temperature ranging from-70°C to 20°C during a time ranging from 5 minutes to 1 hour. The sulfonate obtained is then transformed *"in situ*" to the sulfonyl chloride of formula VII by contacting with *N*-chlorosuccinimide at a temperature ranging from 0°C to 70°C.

The sulfonamide derivatives of formula I are then prepared from the corresponding above mentioned sulfonyl chloride V or VII, by reaction either with a corresponding cyclic amine, e.g. a piperazine or piperidine derivative of the general formula VIII or IX. 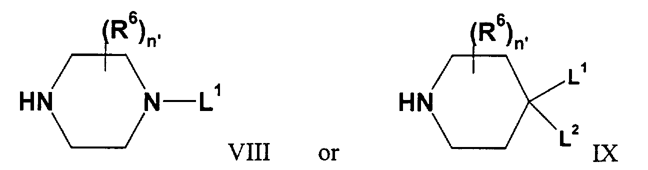 whereby L¹ and L² are as above defined.

The amines of formula VIII or IX are either commercially available compounds or compounds that can be prepared by known procedures.
Typically, piperazines of type VIII can be prepared upon using conventional methods known by a person skilled in the art.
For L¹ and/or L² = aryl, suitable methods of preparation are described in *Tetrahedron Lett.* **1996**, *37*, 8487-8488 and references cited therein.
For L¹ and/or L² = aryl C₁-C₆ alkyl, a further preferred method is the reaction of the corresponding piperazine or mono-*N*-protected piperazine with compounds of formula X 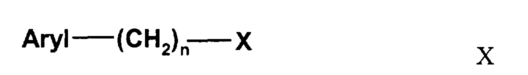 wherein X is Cl, Br, I, OTs, OMs

The reaction is generally conducted in the presence of a base such as triethylamine, diisopropylethylamine, potassium carbonate and the like in solvent such as N,N-dimethylformamide, dimethylsulfoxide, N-methylpyrrolidone, ethanol, acetonitrile at a temperature from about 0° to about 100°C.

For L¹ and/or L² = -C(S)-, a further preferred method is the conversion of compounds of type XI using the Lawesson's reagent which allows the transformation of an amide into a thioamide group as described in *Bull. Soc. Chim. Belgium*, **1978,** *87,* 229.

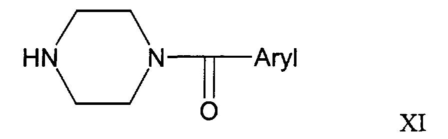

The sulfonamides of formula I are readily prepared by contacting the sulfonyl chlorides V with an amine of formula VIII in the presence of a suitable base to scavenge the acid generated during the reaction. Suitable bases include, by way of examples, triethylamine, diisopropylethylamine, N-methylmorpholine and the like. The reaction is preferably conducted in solvent such as N,N-dimethylformamide, dimethylsulfoxide, N-methylpyrrolidone, ethanol, acetonitrile at a temperature from about 0° to about 100°C. Alternatively, the sulfonamide derivatives of formula I are readily prepared from the corresponding sulfonyl chloride V or VII, by reaction with a piperidine of general formula IX.

Piperidines of formula IX are either commercially available compounds or compounds that can be prepared by known procedures.

Typically, piperidines of type IX can be prepared using conventional methods known by one skilled in the art and described by way of examples in *J. Pharm. Sci.* **1972,** *61,* 1316; *J. Heterocyclic. Chem.,* **1986,** *23,* 73; *Tetrahedron Lett.,* **1996,** *37,* 1297, US 5106983, WO/9113872 and WO/9606609.

### Preferred methods of obtention of piperidines of formula IX are the following :

For L¹ = H and L² = (CH₂)n-Aryl wherein n = 0,1,2; addition of an organometallic species such as Ar³(CH₂)ₙLi or Ar³(CH₂)ₙMgBr on mono-protected 4-piperidone followed by reduction of the so-formed double bound which allows the formation of compounds of type IX.

For L² = -NR-(CH₂)n-Aryl wherein n = 0,1,2, a preferred method is the reductive amination of 4-piperidone with amines of type Aryl-(CH₂)n-NR-H.
A further preferred method in the case where n = 0 is a "Mitsunobu type" coupling between an activated aniline of type XII with mono-N-protected 4-piperidol as described in *Tetrahedron Lett.* **1995,** *36,* 6373-6374.

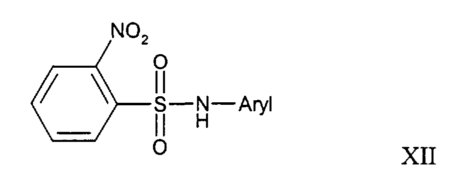

Deprotection of the sulfamino group is then carried out using thiophenol in the presence of potassium carbonate.

For L² = -NR³'C(O)R³, -NR³'C(O)NR³'R³, NR³'SO₂-R³, a preferred method of synthesis of compounds of formula IX is the reaction of commercially available N-BOC-4-aminopiperidine with respectively acyl chlorides, isocyanates and sulfonyl chloride under classical conditions very well known by one skilled in the art.

When L² = -CO-Aryl, compounds of formula IX are readily prepared by contacting well chosen aromatic or heteroaromatic rings with intermediate of type XIII 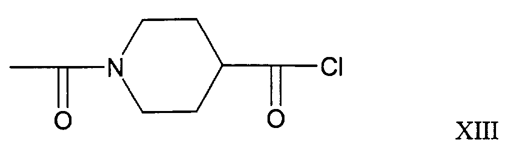 in the presence of a Lewis acid such as aluminum trichloride or titanium tetrachloride in a polar aprotic solvent such as dichloromethane. Intermediate XIII can be easily obtained by first acetylation of piperid-4-yl carboxylic acid and their formation of the acyl chloride by treatment with thionyl chloride.

The sulfonamides of formula I are readily prepared by contacting the sulfonyl chloride V with an amine of formula IX in the presence of a suitable base to scavenge the acid generated during the reaction. Suitable bases include, by way of examples, triethylamine, diisopropylethylamine, N-methylmorpholine and the like. The reaction is preferably conducted in solvent such as N,N-dimethyformamide, dimethylsulfoxide, N-methylpyrrolidone, ethanol, acetonitrile at a temperature from about 0° to about 100°C.

The sulfonamides of formula XIV are readily prepared by contacting the sulfonyl chloride VII with an amine of formula VIII or IX in the presence of a suitable base to scavenge the acid generated during the reaction. Suitable bases include, by way of examples, triethylamine, diisopropylethylamine, N-methylmorpholine and the like. The reaction is preferably conducted in solvent such as N,N-dimethyformamide, dimethylsulfoxide, N-methylpyrrolidone, ethanol, acetonitrile at a temperature from about 0° to about 100°C. The use of sulfonyl chloride of type VII leads to amines that have to be deprotected using well known methods by one skilled in the art to afford amine of general formula XIV 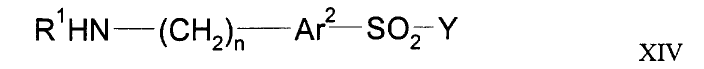 wherein R¹, Ar², Y and n are as above defined.

Derivatives of type XIV are then acylated according to described methods for the preparation of amides by condensation of amines with acid chlorides or carboxylic acids in the preferred conditions described above leading to compounds of general formula I In the particular case of compounds of general formula I where Y represents a piperazine derivative, an alternative method of preparation which has also to be considered as part of this invention, said method of preparation consisting in the condensation of a piperazine derivative of formula XV 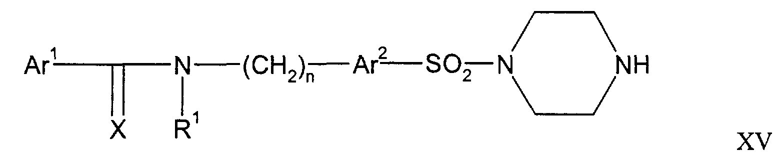 with electrophiles L¹ which will be chosen depending on the nature of L¹ (see the above definition of L¹, L²). Procedures and methods to perform these types of condensation are well-known and have been well described on various synthesis of N-substituted piperazine derivatives.

If the above set out general synthetic methods are not applicable for the obtention of compounds of formula I, suitable methods of preparation known by a person skilled in the art should be used. For example, when Ar² is phenyl, one should start from commercially available 4-cyanophenyl sulfonyl chloride and applies conventional methods known by a person skilled in the art to reach sulfonamide derivatives of formula I.

A final aspect of the present invention is related to the use of the compounds according to formula I for the modulation of the JNK function, or signaling pathways, the use of said compounds for the preparation of pharmaceutical compositions for the modulation of the JNK pathway as well as the formulations containing the active compounds according to formula I. Said modulation of the JNK pathway is viewed as a suitable approach of treatment for various disorders. When employed as pharmaceuticals, the sulfonamide derivatives of the present invention are typically administered in the form of a pharmaceutical composition. Hence, pharmaceutical compositions comprising a compound of formula I and a pharmaceutically acceptable carrier, diluent or excipient therefore are also within the scope of the present invention. A person skilled in the art is aware of a whole variety of such carrier, diluent or excipient compounds suitable to formulate a pharmaceutical composition. Also, the present invention provides compounds for use as a medicament. In particular, the invention provides the compounds of formula I for use as JNK inhibitor, notably of JNK1 and/or JNK2 and/or JNK3, for the treatment of disorders of the immune as well as the neuronal system of mammals, notably of humans, either alone or in combination with other medicaments.

The compounds of the invention, together with a conventionally employed adjuvant, carrier, diluent or excipient may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, or in the form of sterile injectable solutions for parenteral (including subcutaneous use). Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed.

When employed as pharmaceuticals, the sulfonamides derivatives of this invention are typically administered in the form of a pharmaceutical composition. Such compositions can be prepared in a manner well known in the pharmaceutical art and comprise at least one active compound. Generally, the compounds of this invention are administered in a pharmaceutically effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

The pharmaceutical compositions of these inventions can be administered by a variety of routes including oral, rectal, transdermal, subcutaneous, intravenous, intramuscular, and intranasal. Depending on the intended route of delivery, the compounds are preferably formulated as either injectable or oral compositions. The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampoules or syringes of the liquid compositions or pills, tablets, capsules or the like in the case of solid compositions. In such compositions, the sulfonamide compound is usually a minor component (from about 0.1 to about 50% by weight or preferably from about 1 to about 40% by weight) with the remainder being various vehicles or carriers and processing aids helpful for forming the desired dosing form.

Liquid forms suitable for oral administration may include a suitable aqueous or nonaqueous vehicle with buffers, suspending and dispensing agents, colorants, flavors and the like. Solid forms may include, for example, any of the following ingredients, or compounds of a similar nature: a binder such as microcrystalline cellulose, gum tragacanth or gelatine; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Injectable compositions are typically based upon injectable sterile saline or phosphate-buffered saline or other injectable carriers known in the art. As above mentioned, the sulfonamide compound of formula I in such compositions is typically a minor component, frequently ranging between 0.05 to 10% by weight with the remainder being the injectable carrier and the like.

The above described components for orally administered or injectable compositions are merely representative. Further materials as well as processing techniques and the like are set out in Part 8 of *Remington's Pharmaceutical Sciences,* 17^{th} Edition, 1985, Marck Publishing Company, Easton, Pennsylvania, which is incorporated herein be reference. The compounds of this invention can also be administered in sustained release forms or from sustained release drug delivery systems. A description of representative sustained release materials can also be found in the incorporated materials in *Remington's Pharmaceutical Sciences.*

In the following the present invention shall be illustrated by means of some examples which are not construed to be viewed as limiting the scope of the invention.

### Examples

### Example 1: 5-{[(3-methoxybenzoyl)amino]methyl}-2-[(4-{3-[(trifluoromethyl)sulfonyl]anilino}piperidin-1-yl)sulfonyl]thiophene-3-carboxylic acid 1

### Diallyl-thiophen-2-ylmethylamine 1a

A solution of 2-aminomethylthiophene (51.4 g, 956 mmol) and *i*-Pr₂NEt (140 g, 1081 mmol) in CH₂Cl₂ (11) was placed in a 3-1 flask equipped with a condenser and an efficient magnetic agitation. Allyl bromide (115.7 g, 454 mmol) was added, whereupon the moderately exothermic reaction spontaneously reached the reflux temperature after 2 h. The mixture was stirred overnight (16 h), washed (NaHCO₃ sat.; brine), dried (MgSO₄), and concentrated. The resulting oil was filtered over silica gel (EtOAc:hexane 1:4). The filtrate was concentrated and the filtration was repeated to afford 70.3 g (80%) of the title diallylamine as a brown-yellow oil, clean by NMR: ¹H NMR (CDCl₃) δ 7.25 (br. d, *J*= 5.9 Hz, 1H), 6.98 (br. dd, *J*= 5.1, 2.8 Hz, 1H), 6.94-6.92 (m, 1H), 5.99-5.86 (m, 2H), 5.29-5.18 (m, 4H), 3.85 (s, 2H), 3.16 (dd, *J*= 6.3, 0.9 Hz, 4H).

### 5-Diallylaminomethyl-thiophene-2-sulfonyl chloride 1b

A solution of the allyl-protected thiophene **1a** (6.2 g, 32.1 mmol) in Et₂O was cooled to - 70°C by means of an acetone/dry ice bath. A solution of *t*-BuLi in pentane (21.38 ml, 1.5M, 32.1 mmol) was added over 2 min whereupon the internal temperature momentarily rose to -50°C and the mixture turned orange. After 10 min., SO₂ was bubbled for 2 min, which led to the immediate formation of a thick precipitate. The reaction was allowed to reach 0°C, and a suspension of NCS (4.63 g, 32.1 mmol) in THF (20 ml) was added, whereupon the slurry turned purple. After 45 min at r.t., the mixture was filtered over SiO₂, eluting with EtOAc. Evaporation, dilution with EtOAc:hexane 1:5 and filtration over SiO₂ gave 5.0 g (53%) of the title sulfonyl chloride **1b** as a pale brown oil which was used without further purification.

### N,N-Diallyl-N-{[5-(1,4-dioxa-8-azaspiro[4.5]dec-8-ylsulfonyl)thien-2-yl]methyl}amine 1c

Procedure A (from the isolated sulfonyl chloride **1b**). A solution of **1b** (5.84 g, 20 mmol) in CHCl₃ was cooled to 0°C, and treated with 1,4-dioxa-8-azaspiro[4,5]decane (2.8 ml, 22 mmol) and Et₃N (4.2 ml, 30 mmol), and warmed to 23 °C for 10 min. Dilution with EtOAc (100 ml), standard work-up (NaHCO₃ sat.; brine; MgSO₄) and chromatography (EtOAc:cyclohexane 1:2) gave 7.57 g (95%) of the title sulfonamide as a colourless oil.

Procedure B (from **1a,** without isolation of the sulfonyl chloride **1b).** A solution of the allyl-protected thiophene **1a** (29.1 g, 150 mmol) in Et₂O (440 g, 617 ml) was placed in a 1-l three-necked flask (thermometer; argon; septum or SO₂ inlet) and cooled to - 74°C by means of an acetone/dry ice bath. A solution of *t*-BuLi in pentane (100 ml, 1.5M, 150 mmol) was added over 5 min whereupon the internal temperature momentarily rose to -64°C and the mixture turned pink. After 20 min., SO₂ (20 g, 312 mmol) was bubbled over 15 min. The SO₂ consumption was best monitored by placing the SO₂ bottle on a scale during the reaction The reaction mixture, which had turned to a thick, white wax was allowed to warm to room temperature over 2h. A suspension of NCS (30 g, 226 mmol) was added, and stirring was continued overnight, whereupon the slurry turned purple. The mixture was filtered (fritted glass), and the precipitate was carefully washed with CH₂Cl₂ (2 x 300 ml). The combined organic layers were cooled to 0°C under Ar, and treated with a solution of 1,4-dioxa-8-azaspiro[4,5]decane (27.8 g, 194 mmol) and triethylamine (19.7 g, 194 mmol) in CH₂Cl₂ (200 ml). After 1 h, the mixture was washed (NaHCO₃ sat.; brine), dried (MgSO₄), and concentrated to afford 53 g (83%) of the title sulfonamide as yellow oil: ¹H NMR (CDCl₃) δ7.36 (d, *J*= 3.8 Hz, 1H), 6.90 (br. d, *J*= 3.4 Hz, 1H), 5.92-5.79 (m, 2H), 5.33-5.16 (m, 4H), 3.93 (s, 4H), 3.78 (s, 2H), 3.21 (t, 5.7 Hz, 4H), 3.13 (d, 6.2 Hz, 4H), 1.81 (t, 5.7 Hz, 4H).

### Ethyl 5-[(diallylamino)methyl]-2-(1,4-dioxa-8-azaspiro[4.5]dec-8-ylsulfonyl)thiophene-3-carboxylate 1d

A solution of the sulfonamide **1c** (3.36 g, 8.43 mmol) in THF (120 ml) was cooled to-78°C and treated with *t*-BuLi (7.0 ml, 1.5M in hexane, 10.5 ml). After 5 min, the mixture was canulated into a cooled (-100°C; acetone/liquid N₂) solution of ethyl chloroformate (6.45 ml, 67.5 mmol) in THF (60 ml). The reaction mixture was allowed to warm to -30°C over 2h, and then to 23°C overnight. The mixture was concentrated °C over 2h, and then to 23°C overnight. The mixture was concentrated on a rotary evaporator and diluted with EtOAc (250 ml). Standard work-up (H₂O; brine; MgSO₄) and two chromatographies (EtOAc:cyclohexane 1:4) afforded 1.48 (37%) of the title ethyl ester: ¹H NMR (DMSO-*d6*) δ7.36 (d, 1H), 5.98-5.82 (m, 2H), 5.32-5.17 (m, 4H), 4.33 (q, *J*= 7.1 Hz, 2H), 3.92 (s, 4H), 3.85 (s, 2H), 3.32 (dd, *J*≈ 6.0, 5.0 Hz, 4H), 3.17 (d, *J*= 6.0 Hz, 4H), 1.74 dd, *J* ≈ 6.0, 5.0 Hz, 4H), 1.33 (t, *J*= 7.2 Hz, 3H).

### Ethyl 2-(1,4-dioxa-8-azaspiro[4.5]dec-8-ylsulfonyl)-5-{[(3-methoxybenzoyl)amino]methyl}thiophene-3-carboxylate 1e

A solution of the ethyl ester **1d** (1.47 g, 3.12 mmol) and NDMBA (1.07 g, 6.87 mmol) in CH₂Cl₂ (30 ml) was degassed by bubbling argon and sonicating. Then, Pd(PPh₃)₄ (216 mg, 0.187 mmol) was added and the mixture was stirred at 23°C. After 2h, the mixture was cooled to-50°C, treated with Et₃N (525 ul, 3.76 mmol) and 3-(methoxy)benzoyl chloride (300ul, 2.13 mmol), and warmed to r.t. over 30 min. Dilution with EtOAc, standard work-up (H₂O; NaHCO₃ sat.; brine; MgSO₄) and chromatography (EtOAc:cyclohexane 1:1) afforded 1.0 g (61%) of the title 3-methoxybenzamide: ¹H NMR (DMSO-*d6*) 9.29 (t, *J=* 5.8 Hz, 1H), 7.49-7.34 (m, 4H), 7.12 (ddd, *J=* 7.9, 2.6, 1.0 Hz, 1H), 4.66 (d, *J*= 5.7 Hz, 2H), 4.27 (q, *J=* 7.2 Hz, 2H), 3.84 (s, 4H), 3.80 (s, 3H), 3.24 (dd, *J*≈ 6.0, 5.0 Hz, 4H), 1.67 (dd, *J*≈ 6.0, 5.0 Hz, 4H), 1.26 (t, *J=* 7.0 Hz, 3H). M/Z APCI: 525 (M + 1), 523 (M-1).

### Ethyl 5-{[(3-methoxybenzoyl)amino]methyl}-2-[(4-oxopiperidin-1-yl)sulfonyl]thiophene-3-carboxylate 1f

A solution of the spiroketal **1e** (500 mg, 0.953 mmol) in acetone (5 ml) was treated with HCl 1N (2.5 ml) for 18 h at 48°C. Dilution with EtOAc and standard work-up (H₂O; NaHCO₃ sat.; brine; MgSO₄) gave 425 mg of a 9:1 mixture of the desired title ketone (83%) and of unreacted starting material (9%) (single spot by TLC). ¹H NMR (CDCl₃) 7.37-7.35 (m, 1H), 7.33-7.29 (m, 3H), 7.05 (ddd, *J*= 7.7, 2.6, 1.7 Hz, 1H), 6.81 (t, *J*= 5.8 Hz, 1H), 4.74 (d, *J*= 6.1 Hz, 2H), 4.31 (q, *J=* 7.1 Hz, 2H), 3.83 (s, 3H), 3.70 (t, *J=* 6.1 Hz, 4H), 2.52 (t, *J=* 6.2 Hz, 4H), 1.34 (t, *J=* 7.1 Hz, 3H). M/Z APCI: 481 (M + 1), 479 (M-1).

### Ethyl 5-{(3-methoxybenzoyl)amino]methyl)}-2-[(4-{3-[(trifluoromethyl)sulfonyl]anilino}piperidin-1-yl)sulfonyl]thiophene-3-carboxylate 1g

A suspension of the crude ketone **1f** (425 mg, 0.803 mmol), 3-(trifluoromethylsulfonyl)aniline (287 mg, 1.27 mmol), and 3 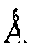 powdered MS (3 g) in dry tetrachloroethylene (15 ml) was heated to reflux for 17h under strictly anhydrous conditions. The mixture was cooled to 23°C, and finely powdered NaBH(OAc)₃ (1.2 g) was added. The stirring was continued for 2.5 d.. Dilution with EtOAc, standard work-up (NaHCO₃ sat.; brine; MgSO₄) and chromatography (EtOAc:cyclohexane 1:1.5 → 2:1) afforded 167 mg (35%) of a mixture of the starting ketone **1f** and spiroketal **1e,** and 216 mg (39%) of the title anilinopiperidine. ¹H NMR (DMSO-*d6*) 9.16 (t, *J*= 5.8 Hz, 1H), 7.38-7.23 (m, 5H), 6.97-7.08 (m, 4H), 6.42 (d, *J*= 7.9 Hz, 1H), 4.53 (d, *J*= 5.7 Hz, 2H), 4.15 (q, *J*= 7.0 Hz, 2H), 3.68 (s, 3H), 3.53 (dm, *J*= 10.4 Hz, 2H), 3.60-3.43 (m, 1H), 2.81 (br. t, *J* = 10.6 Hz, 2H), 1.84 (dm, *J* ≈ 11.3 Hz, 2H), 1.35-1.20 (m, 2H), 1.15 (t, *J* = 7.0 Hz, 3H). M/Z APCI: 690 (M + 1), 688 (M-1).

### 5-{[(3-Methoxybenzoyl)amino]methyl}-2-[(4-{3-[(trifluoromethyl)sulfonyl]anilino}piperidin-1-yl)sulfonyl]thiophene-3-carboxylic acid 1

A solution of the ethyl ester **1g** (40 mg, 0.058 mmol) in MeOH (4 ml) was treated with NaOH 2M (0.8 ml) for 2 h at 45°C. The mixture was diluted with EtOAc, washed (NH₄Cl aq.; H₂O; brine), dried (MgSO₄), concentrated to 2 ml, and filtered over celite, eluting with EtOAc. Evaporation gave 40 mg (96%) of the title acid . M/Z APCI: 662 (M+1), 660 (M-1), 616 (M-CO₂-1). Anal. HPLC: R.t = 6.55 min (method a).

The following compounds was prepared according to the above described procedure by replacing 3-(trifluoromethylsulfonyl) aniline with octylamine in the reductive amination step.

The following table provides HPLC data and mass spectroscopy data of the mentioned examples. ¹,².

| **Exemple** | **Name** | **Rt HPLC** | **Purity** | **Gradient HPLC** | **Mass M+1** | **Mass M** |
|---|---|---|---|---|---|---|
| **2** | 5-{[(3-methoxybenzoyl)amino]methyl}-2-{[4-(octylamino)piperidin-1-yl]sulfonyl}thiophene-3-carboxylic acid | 4.58 | 90.1 | a | 567 | 565 |

¹ HPLC conditions: C8 Symmetry a- MeCN, 0.09%TFA, 0 to 100% (10min)
² Mass spectrum APCI

### Exemple 3: N-(2-hydroxyethyl)-5-{[(3-methoxybenzoyl)amino]methyl}-2-[(4-{3-[(trifluoromethyl)sulfonyl]anilino}piperidin-1-yl)sulfonyl]thiophene-3-carboxamide

A solution of the ethyl ester **1g** (10 mg, 0.015 mmol) and ethanolamine (0.1 ml) in MeOH (1 ml) was heated to reflux for 8 h and concentrated to dryness to afford the title amide in nearly quantitative yield. M/Z APCI : 705 (M+1), 703 (M-1). Anal. HPLC: R.t = 6.14 min (method a).
The following compounds were prepared according to the above described procedure (exemple 2) by replacing ethanolamine with hydrazine, aqueous ammonia or N,N'-dimethylaminoethylene diamine
The following table provides HPLC data and mass spectroscopy data of the mentioned examples

| **Example** | **Name** | **Rt HPLC** | **Purity** | **Gradient HPLC** | **Mass M+1** | **Mass M** |
|---|---|---|---|---|---|---|
| 4 | N-({4-(hydrazinocarbonyl)-5-[(4-{3-[(trifluoromethyl)sulfonyl]anilino}piperidin-1-yl)sulfonyl]thien-2-yl}methyl)-3-methoxybenzamide | 5.67 | 79.0 | a | 520 | 518 |
| 5 | 5-{[(3-methoxybenzoyl)amino]methyl}-2-[(4-{3-[(trifluoromethyl)sulfonyl]anilino}piperidin-1-yl)sulfonyl]thiophene-3-carboxamide | 5.63 | 84.4 | a | 661 | 659 |
| 6 | N-[2-(dimethylamino)ethyl]-5-{[(3-methoxybenzoyl)amino]methyl}-2-[(4-{3-[(trifluoromethyl)sulfonyl]anilino}piperidin-1-yl)sulfonyl]thiophene-3-carboxamide | 4.85 | 98.0 | a | 732 | 730 |

### Exemple 7: N-({4-(hydroxymethyl)-5-[(4-{3-[(trifluoromethyl)sulfonyl]anilino}piperidin-1-yl)sulfonyl]thien-2-yl}methyl)-3-methoxybenzamide 7

A solution of the carboxylic acid (19 mg, 0.029 mmol) was dissolved in borane-THF complex (1M in THF, 1ml, 1 mmol), and the solution was stirred for 30 min at 23°C. The reaction was quenched with water (1 ml, fizz!), diluted with EtOAc (10 ml), dried (MgSO₄), concentrated, and chromatographed (EtOAc:cyclohexane 1:2 → 2:1) to give 11.1 mg (60%) of the title alcohol. M/Z APCI : 648 (M+1), 646 (M-1). Anal. HPLC: R.t = 6.43 min (method a).

### Example 7 : Preparation of a pharmaceutical formulation

The following formulation examples illustrate representative pharmaceutical compositions according to the present invention being not restricted thereto.

### Formulation 1 - Tablets

A sulfonamide compound of formula I is admixed as a dry powder with a dry gelatin binder in an approximate 1:2 weight ration. A minor amount of magnesium stearate is added as a lubricant. The mixture is formed into 240-270 mg tablets (80-90 mg of active sulfonamide compound per tablet) in a tablet press.

### Formulation 2 - Capsules

A sulfonamide compound of formula I is admixed as a dry powder with a starch diluent in an approximate 1:1 weight ratio. The mixture is filled into 250 mg capsules (125 mg of active sulfonamide compound per capsule).

### Formulation 3 - Liquid

A sulfonamide compound of formula I (1250 mg), sucrose (1.75 g) and xanthan gum (4 mg) are blended, passed through a No. 10 mesh U.S. sieve, and then mixed with a previously prepared solution of microcrystalline cellulose and sodium carboxymethyl cellulose (11:89, 50 mg) in water. Sodium benzoate (10 mg), flavor, and color are diluted with water and added with stirring. Sufficient water is then added to produce a total volume of 5 mL.

### Formulation 4 - Tablets

A sulfonamide compound of formula I is admixed as a dry powder with a dry gelatin binder in an approximate 1:2 weight ratio. A minor amount of magnesium stearate is added as a lubricant. The mixture is formed into 450-900 mg tablets (150-300 mg of active sulfonamide compound) in a tablet press.

### Formulation 5 - Injection

A sulfonamide compound of formula I is dissolved in a buffered sterile saline injectable aqueous medium to a concentration of approximately 5 mg/ml.

### Example 8 : Biological assays

### Biological Results

The activities of the sulfonamide derivatives claimed in the formula I were assessed using the above described *in vitro* and *in vivo* biological assays.

**JNK 2 and 3 in vitro assay:** JNK3 assay is performed in 96 well MTT plates, by incubation of 0.5 µg of recombinant, pre-activated GST-JNK3 or GST-JNK2 with 1 µg of recombinant, biotinylated GST-c-Jun and 2 µM ³³γ-ATP (2 nCi/µl), in the presence or absence of sulfonamide inhibitors if formula I and in a reaction volume of 50 µl containing 50 mM Tris-HCl, pH 8.0; 10 mM MgCl₂; 1 mM Dithiothreitol, and 100 µM NaVO₄. The incubation is performed for 120 min. at R.T and stopped upon addition of 200 µl of a solution containing 250 µg of Streptavidine-coated SPA beads (Amersham, Inc.)*, 5 mM EDTA, 0.1% Triton X-100 and 50 µM ATP, in phosphate saline buffer. After incubation for 60 minutes at RT, beads are sedimented by centrifugation at 1500 x g for 5 minutes, resuspended in 200 µl of PBS containing 5 mM EDTA, 0.1% Triton X-100 and 50 µM ATP and the radioactivity measured in a scintillation β counter, following sedimentation of the beads as described above.

| *Example* | *JNK3* |
|---|---|
| **1** | 0.01 |
| **4** | 0.100 |
| **6** | 0.07 |

The values indicated in respect of refer to the IC₅₀ (µM), i.e. the amount necessary to achieve 50% inhibition of said target. Said values show a considerable potency of the sulfonamide compounds with regard to JNK3.

### Sympathetic Neuron Culture and Survival Assay

Sympathetic neurons from superior cervical ganglia (SCG) of new-born rats (p4) are dissociated in dispase, plated at a density of 10⁴ cells/cm² in 48 well MTT plates coated with rat tail collagen, and cultured in Leibowitz medium containing 5% rat serum, 0.75 µg/mL NGF 7S (Boehringer Mannheim Corp., Indianapolis, IN.) and arabinosine 10⁵M. Cell death is induced at day 4 after plating by exposing the culture to medium containing 10 µg/mL of anti NGF antibody (Boehringer Mannheim Corp., Indianapolis, IN.) and no NGF or arabinosine, in the presence or absence of sulfonamide inhibitors. 24 hours after cell death induction, determination of cell viability is performed by incubation of the culture for 1 hour, at 37°C in 0.5 mg/mL of 3-(4,5-dimethylthiazol-2-yl)2,5 diphenyl tetrazolium bromide (MTT). After incubation in MTT cells are resuspended in DMSO, transferred to a 96 MTT plate and cell viability is evaluated by measuring optical density at 590 nm.

The results of this assay with various test compounds demonstrate that compounds of Formula I are rescuing neurons from cells death (% neurons alive between 10 and 80)

### Il-2 Release Assay:

Jurkat cells, a human T cell leukemia cell line (American Type Culture Collection # TIB 152) were cultured in RPMI 1640 medium (Gibco, BRL) supplemented with 10% of heat-activated FCS, Glutamine and Penstrep. The cell suspension in the medium is diluted to give 2.10⁶ cells/mL. The cells were plated (2.10⁵ cells/well) on a 96-well plate containing different concentration of test compound (final concentration of compounds, 10, 3, 1, 0.3, 0.1 µM). This mixture is incubated 30 minutes at 37°C in a humidified CO₂ atmosphere. Cells were then treated with 10 ul PMA + Ionomycine (0.1 µM and 1 µM final concentration) in all wells except negative control. In wells without compounds, 10 µl of RPMI 2% DMSO (=0.1% final) is added. Cells are incubated 24 hours at 37°C and then the supernatant harvested (freeze at -20°C if not used the same day) prior to performing IL-2 ELISA test on the supernatant.

### IL-2 ELISA Assay:

IL-2 release into the medium by PMA+Iono-stimulated Jurkat cells, in presence or absence of test compounds is assayed by ELISA. Following the procedure described below
***Solutions***
   Wash buffer: PBS- Tween 0.05%
   Diluent: PBS- Tween 0.05%
   Substrate solution: Citric acid 0.1M/Na₂HPO₄ 0.1M
   Stop solution: H₂SO₄ 20%
***Matched Antibody pairs/standard:***
   From R&D Systems
   Monoclonal anti-human IL-2 antibody (MAB602) (capture)
   Biotinylated anti-human IL-2 antibody (BAF202) (detection)
   Recombinant human IL-2 (202-IL-010) (standard)
***Plate preparation***
   Transfer 100 µl capture antibody diluted in PBS at 5 µg/mL into a 96 well ELISA plate and incubate overnight at room temperature.
   Aspirate each well and wash 3 times with Wash buffer. After the last wash, damp the plate.
   1. Saturate with 200 µl PBS-10% FCS. Incubate 1 hour at room temperature.
   2. Repeat the wash step 2.
***Assay procedure***
   1. Add 100 µl of sample or standard (2000, 1000, 500, 250, 125, 62.5, 31.25pg/mL) and incubate 2 hours at room temperature.
   2. Wash 3 times.
   3. Add 100 µl of biotinylated anti-human IL-2 at 12.5 ng/mL. Incubate 2 hours at room temperature.
   4. Wash 3 times.
   5. Add 100 µl streptavidin-HRP (Zymed #43-4323) at 1:10'000. Incubate 30 minutes at room temperature.
   6. Wash 3 times
   7. Add 100 µl substrate solution (citric acid/ Na₂HPO₄ (1:1) + H₂O₂ 1:2000 + OPD). Incubate 20-30 minutes at room temperature.
   8. Add 50 µl of stop solution to each well.
   9. Determine optical density using a microtiter plate reader set to 450 nm with correction at 570 nm.

The result of this assay shows that various test compounds decrease the production of IL-2 of more than 30% at 3 µM.

### C-Jun Reporter Assay

### Cell culture

Hlr c-Jun HeLa cells are cultured in DMEM High Glc supplemented with 10% FCS (Sigma), 2mM Glutamine (Gibco), P/S, Hygromycin b 100µg/mL and G418 250µg/mL

### Cell culture preparation

### Cell Banks

The cells are stored frozen in cryotubes under liquid nitrogen, as 1.8 mL volumes of cell suspension in culture medium containing 10% dimethyl sulfoxide.
Cells are kept in culture for no more than 20 passages.

### Cell culture thawing

When necessary, frozen vials of cells are thawed rapidly at 37°C in a water bath by gently swirling up to semi-complete thawing. Then the cell suspension are added to 10 mL of culture medium.
The cell suspension is then centrifuged for 5 minutes at 1200 rpm, the supernatant is removed and the cell pellet reconstituted in the medium and add to a 175 cm² flask containing 25 mL medium. The flasks are incubated at 37° C in an atmosphere of 5 % CO₂.

### Cell passage

The cells are serially subcultured (passaged) when 80% confluent monolayers have been obtained.
The medium of each flask is removed and the monolayer is washed with 10-15 mL of phosphate buffer solution (PBS).

Trypsin-EDTA solution is added to the cell monolayer, incubated at 37° C and tapped gently at intervals to dislodge the cells. Complete detachment and disaggregation of the cell monolayer is confirmed by microscopy examination. The cells are then resuspended in 10 mL of complete medium and centrifuged for 5 minutes at 1200 rpm.
The supernatant are discarded, the cells are resuspended in culture medium and diluted 1/5 in 175 cm² flasks.

### Day 0 morning

### Prepare cells for transfections

The cells from flasks of near-confluent cultures are detached and disaggregated by treatment with trypsin as described above.
The cells are resuspended in culture medium and counted.
The cell suspension are diluted with medium to give about 3.5x10⁶ cells/mL and 1mL µl of cell suspension are put onto 2 10cm culture dishes containing 9 mL of culture medium.
The plates are incubated at 37° C in a humidified atmosphere of 5 % CO₂ in air

### Day 0 evening

### Transfections

| | |
|---|---|
| Control | 0.2µg pTK Renilla, 5.8µg pBluescript KS, 500µl OPTIMEM (GIBCO), 18µl Fugene 6 |
| Induced | 0.1µg pMEKK1, 0.2µg pTK Renilla, 5.7µg pBluescript KS, 500µl OPTIMEM (GIBCO), 18µl Fugene 6 30' RT |

The transfection mixture is added to the plated cells. The plates are incubated over night at 37° C in a humidified atmosphere of 5 % CO₂ in air

### Day 1

A 96 wells plate containing 100 µl of culture medium per well is prepared
Negative control (vehicle): 2µl of DMSO is added to the 100µl(in triplicate).
Compound : 2 µl of Hit compound stock dilution are added to the 100µl(in triplicate).
The transfected cells are trypsinised and re-suspend in 12 mL of culture medium. 100µl of the dilution are added to each of the 96 wells plate.
The plate is incubated over night at 37° C in a humidified atmosphere of 5 % CO₂ in air

### Hit compound dilutions

Hit compound stock concentrations are the following:
3, 1 and 0.1mM in 100% DMSO.

### Day 2

### Test procedure

### Dual-Luciferase™ Reporter Assay System (Promega)

The medium is removed from the plate and the cells washed two times with 100µl PBS Completely remove the rinse solution before applying PLB reagent. Dispense into each culture well 5µl of 1X PLB. Place the culture plates on a rocking platform or orbital shaker with gentle
rocking/shaking to ensure complete and even coverage of the cell monolayer with 1X PLB. Rock the culture plates at room temperature for 15 minutes. Transfer 20 µl of the lysate into a white opaque 96 wells plate. Read in a luminometer.
- Inject 50µl of Luciferase Assay Reagent II wait 5", read 10"
- Inject 50µl of Stop & Glo ® Reagent wait 5", read 10"

### Check RLU Luciferase/RLU Renilla*1000

The result of this assay shows that various test compounds inhibit more than 20% of the activity of JNK at 10 µM.

### LPS induced Endotoxin Shock in Mice

*The ability of the JNK inhibitors described in formula I to significantly reduce the level of inflammatory cytokines induced by LPS challenge was assessed using the following protocol:*

LPS (S. abortus-Galanos Lab.-) was injected (200 µg/kg, i.v.) to Male C57BL/6 to induce endotoxin shock and compounds (0.1, 1, 10 mg/kg) or NaCl (200uM) were injected intravenously (10 mL/kg) 15 min before the LPS challenge. Heparinized blood was obtained from the orbital sinus at different time points after the LPS challenge, and the blood was centrifuged at 9'000 rpm for 10 min at 4° C to collect supernatant for the measurement of cytokines production by mouse ELISA kit such as IFNγ (Duoset R&D Ref. DY485).

### Global Ischemia in Gerbils

*The ability of the JNK inhibitors described in formula I to protect cell death during a stroke event was assessed using the following protocol:*

### -1- METHOD

∗ Surgery
   - Anesthesia: halothane or isoflurane (0.5-4%).
   - Sheaving of the gorge and incision of the skin.
   - The common carotid arteries (left and right) are freed from tissue.
   - Occlusion of the arteries using Bulldog microclamps during 5 min.
   - Disinfection of the surgery plan (Betadine®) and suture of the skin (Autoclip® ou Michel's hooks).
   - Stabulation of the animals under heating lamp until awake.
   - Stabulation of the animals in the animalry in individual cages.
∗ Sacrifice of the animals
   - 7 days after ischemia (Decapitation or overdose of pentobarbital).
   - Sampling of the brain.
∗ Histological parameters
   - Freezing of the brain in isopentane (-20°C)
   - Slicing of the hippocampus using a cryo-microtome (20 µm).
   - Staining with cresyl violet and/or TUNEL method
   - Evaluation of the lesions (in CA1/CA2 subfields of the hippocampus)
      - Gerhard & Boast score modified or
      - Cell counting in the CA1/CA2
∗ Biochemical parameters
   - Microdissection of the cerebral structures
   - Parameters determined: DNA fragmentation, lactate, calcium penetration.
   - Analytical methods: ELISA, colorimetry, enzymology, radiometry.

### -2- TREATMENT

- Administration of the test article or the vehicle: 15 min after reperfusion (5-10 min after the recovery of the anesthesia).
- Standard protocol
50 animals : 5 groups of 10 (group A : control, groups B-D : test article at 3 doses and group E : reference compound (ketamine 3x120 mg/kg, *ip* or Orotic acid 3x300 mg/kg, *ip).*

The test compounds displayed considerable capability to protect from neuronal apoptosis during induced global ischemia.

## Claims

1. Hydrophilic sulfonamide derivatives according to formula I 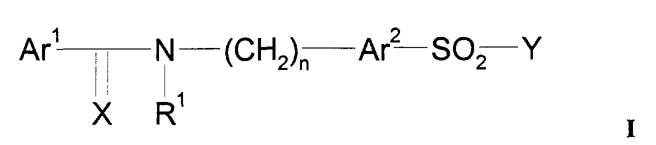 with its geometrical isomers, in an optically active form as enantiomers, diastereomers, as well as in the form of racemates and the pharmaceutically acceptable salts thereof, wherein
Ar¹ is a substituted or unsubstituted aryl or heteroaryl;
Ar² is an aryl or heteroaryl group carrying at least one hydrophilic substituent;
X is O or S, preferably O;
R¹ is hydrogen or a C₁-C₆-alkyl group, or R¹ forms a substituted or unsubstituted 5-6-membered saturated or unsaturated ring with Ar¹;
n is an integer from 0 to 5, preferably between 1-3 and most preferred 1;
Y within formula I is an unsubstituted or a substituted 4-12-membered saturated cyclic or bicyclic alkyl containing at least one nitrogen atom, whereby one nitrogen atom within said ring is forming a bond with the sulfonyl group of formula I thus providing a sulfonamide.

2. A sulfonamide derivative according to claim 1, wherein Y is a piperidine group of the general formula 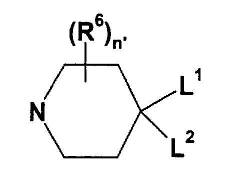 whereby, L¹ and L²are independently selected from each other from the group comprising or consisting of H, substituted or unsubstituted C₁-C₆-aliphatic alkyl, substituted or unsubstituted C₂-C₆-alkenyl, substituted or unsubstituted C₂-C₆-alkynyl, substituted or unsubstituted cyclic C₄-C₈-alkyl optionally containing 1-3 heteroatoms and optionally fused with aryl or heteroaryl; or L¹ and L² are independently selected from the group comprising or consisting of substituted or unsubstituted aryl, substituted or unsubstituted heteroaryl, aryl-C₁-C₆-alkyl, het-eroaryl-C₁-C₆-alkyl, -C(O)-OR³, -C(O)-R³, -C(O)-NR^{3'}R³, -NR^{3'}R³,-NR^{3'}C(O)R³,-NR^{3'}C(O)NR^{3'}R³,-(SO)R³,-(SO₂)R³,-NSO₂R³,-SO₂NR^{3'}R³, with R³, R^{3'} being substituents independently selected from the group comprising or consisting of H, substituted or unsubstituted C₁-C₆-alkyl, substituted or unsubstituted C₂-C₆-alkenyl, substituted or unsubstituted aryl, substituted or unsubstituted heteroaryl, substituted or unsubstituted aryl-C₁-C₆-alkyl, substituted or unsubstituted heteroaryl-C₁-C₆-alkyl;
said aryl or heteroaryl groups being optionally substituted C₁-C₆-alkyl, C₁-C₆-alkoxy, C₂-C₆-alkenyl, C₂-C₆-alkynyl, amino, acylamino, aminocarbonyl, C₁-C₆-alkoxycarbonyl, aryl, carboxyl, cyano, halogen, hydroxy, nitro, sulfonyl, sulfoxy, C₁-C₆-thioalkoxy,
or L¹ and L² taken together form a 4-8-membered, substituted or unsubstituted saturated cyclic alkyl or heteroalkyl group; and
R⁶ is selected from the group comprising or consisting of hydrogen, substituted or unsubstituted C₁-C₆-alkyl, substituted or unsubstituted C₁-C₆-alkoxy, OH, halogen, nitro, cyano, sulfonyl, oxo (=O), and
n' is an integer from 0 to 4, preferably 1 or 2.

3. A sulfonamide derivative according to any of the preceding claims, wherein Ar¹ is an unsubstituted or substituted phenyl.

4. A sulfonamide derivative according to any of the preceding claims, wherein Ar² is a thienyl, pyrrolo or furanyl group with at least one, preferably one, hydrophilic substituent.

5. A sulfonamide derivative according to claim 4, wherein the hydrophilic group is a carboxylic group, a carboxylate, a carboxamide, OH, or an alkyl group carrying an OH-substituent, or a hydrazido carbonyl group.

6. A sulfonamide derivative according to any of the preceding claims, wherein Ar¹ is a phenyl group, X is O, R¹ is hydrogen, n is 1, Ar² is a thienyl group with one hydrophilic substituent.

7. A sulfonamide derivative according to claim 6, wherein Y is 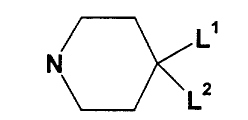
whereby L² is H, L¹ is -NHR³ with R³ being a substituent selected from the group consisting of C₁-C₁₂-alkyl, aryl, heteroaryl, aryl-C₁-C₆-alkyl, heteroaryl-C₁-C₆-alkyl;
said aryl or heteroaryl groups being optionally substituted by halogen, hydroxy, nitro, sulfonyl.

8. A sulfonamide derivative according to any of the preceding claims selected from the following group :
5-{[(3-methoxybenzoyl)amino]methyl }-2-[(4-{3-[(trifluoromethyl)sulfonyl]anilino}piperidin-1-yl)sulfonyl]thiophene-3-carboxylic acid
5-{[(3-methoxybenzoyl)amino]methyl}-2-{[4-(octylamino)piperidin-1-yl]sulfonyl}thiophene-3-carboxylic acid
N-(2-hydroxyethyl)-5-{[(3-methoxybenzoyl)amino]methyl }-2-[(4-{3-[(trifluoromethyl)sulfonyl]anilino}piperidin-1-yl)sulfonyl]thiophene-3-carboxamide
N-({4-(hydrazinocarbonyl)-5-[(4-{3-[(trifluoromethyl)sulfonyl]anilino }piperidin-1-yl)sulfonyl]thien-2-yl}methyl)-3-methoxybenzamide
5-{[(3-methoxybenzoyl)amino]methyl}-2-[(4-{3-[(trifluoromethyl)sulfonyl]anilino}piperidin-1-yl)sulfonyl]thiophene-3-carboxamide
N-[2-(dimethylamino)ethyl]-5-{[(3-methoxybenzoyl)amino]methyl}-2-[(4-{3-[(trifluoromethyl)sulfonyl]anilino}piperidin-1-yl)sulfonyl]thiophene-3-carboxamide
N-({4-(hydroxymethyl)-5-[(4-(3-[(trifluoromethyl)sulfonyl]anilino}piperidin-1-yl)sulfonyl]thien-2-yl}methyl)-3-methoxybenzamide

9. A sulfonamide derivative according to any of the preceding claims , for use as a medicament.

10. Use of a sulfonamide derivative according to any of claims 1 to 8 for the preparation of a pharmaceutical composition for the modulation of the JNK pathway.

11. Use according to claim 10 for the treatment or prevention of disorders associated with the abnormal expression or activity of JNK.

12. Use according to claim 11 for the treatment or prevention of disorders associated with abnormal expression or activity of JNK2 and/or 3.

13. Use of a sulfonamide derivative according to formula I in particular according to any of claims 10 to 12 for the treatment of neuronal disorders including epilepsy; Alzheimer's disease, Huntington's disease, Parkinson's disease; retinal diseases, spinal cord injury, head trauma.

14. Use of a sulfonamide derivative according to formula I in particular according to any of claims 10 to 12 for the treatment of autoimmune diseases including Multiple Sclerosis, inflammatory bowel disease (IBD), rheumatoid arthritis, asthma, septic shock, transplant rejection.

15. Use of a sulfonamide derivative according to formula I in particular according to any of claims 10 to 12 for the treatment of cancer including breast-, colorectal-, pancreatic cancer.

16. Use of a sulfonamide derivative according to formula I in particular according to any of claims 10 to 12 for the treatment of cardiovascular diseases including stroke, arterosclerosis, myocordial infarction, myocordial reperfusion injury.

17. A pharmaceutical composition containing at least one sulfonamide derivative according to any of the claims 1 to 8 and a pharmaceutically acceptable carrier, diluent or excipient thereof.

18. Process for the preparation of a sulfonamide derivative according to any of claims 1 to 8, wherein a sulfonyl chloride V 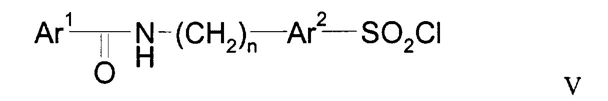 is reacted with a cyclic amide, in particular with an amine VIII 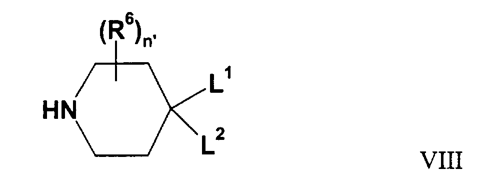 whereby (R⁶)ₙ, L¹ and L² are as above defined.

19. A process according to claim 18, wherein a sulfonyl chloride V is obtainable by
a) coupling an amine of formula II: 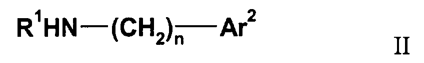 where Ar² and R¹ are as defined above, with an acyl chloride of formula III: 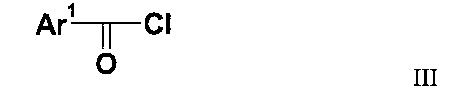 where Ar¹ is as defined above, to provide an amide of formula IV: 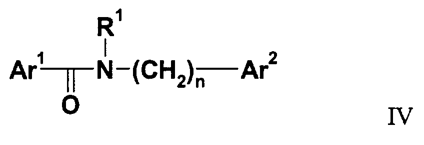
b) sulfonating the amide of formula IV to provide a sulfonyl chloride V
